Europäisches Patentamt

European Patent Office

Office européen des brevets

(19)

(11) Veröffentlichungsnummer: **0 318 703**
**A1**

(12) **EUROPÄISCHE PATENTANMELDUNG**

(21) Anmeldenummer: **88118039.2**

(22) Anmeldetag: **29.10.88**

(51) Int. Cl.⁴: **C12N 15/00**

Claims for the following Contracting States: ES + GR.

(30) Priorität: **03.11.87 DE 3737239**

(43) Veröffentlichungstag der Anmeldung:
**07.06.89 Patentblatt 89/23**

(84) Benannte Vertragsstaaten:
**AT BE CH DE ES FR GB GR IT LI LU NL SE**

(71) Anmelder: **BEHRINGWERKE**
**Aktiengesellschaft**
**Postfach 1140**
**D-3550 Marburg 1(DE)**

(72) Erfinder: **Grundmann, Ulrich, Dr.**
**Am Pfahltor**
**D-3551 Lahntal-Grossfelden(DE)**
Erfinder: **Abel, Karl-Josef, Dr.**
**Am Ziegenberg 6**
**D-3550 Marburg(DE)**
Erfinder: **Küpper, Hans, Dr.**
**Biegenstrasse 39**
**D-3550 Marburg(DE)**

(74) Vertreter: **Meyer-Dulheuer, Karl-Hermann, Dr.**
**et al**
**HOECHST Aktiengesellschaft Zentrale**
**Patentabteilung Postfach 80 03 20**
**D-6230 Frankfurt/Main 80(DE)**

(54) Gentechnische Herstellung von anticoagulatorischem Protein PP4.

(57) Die cDNA, die für PP4 kodiert, wird beschrieben. Mit Hilfe dieser cDNA kann PP4 in prokaryotischen oder eukaryotischen Zellen hergestellt werden.

EP 0 318 703 A1

## Gentechnische Herstellung von anticoagulatorischem Protein PP4

Das anticoagulatorische Protein PP4 ist in der DE-A 33 15 000 (US-A 4,507,229) mit folgenden Parametern beschrieben:
- einer elektrophoretischen Beweglichkeit im Bereich zwischen der von $alpha_1$- und $alpha_2$-Globulinen;
- einem isoelektrischen Punkt von 4,85 ± 0,15;
- einem Sedimentationskoeffeizienten $s_{20, w}^{0}$ von 3,3 ± 0,2S;
- einem im natriumdodecylsulfat (SDS)-haltigen Polyacrylamidgel bestimmten Molekulargewicht von 35 000 ± 5 000;
- einem Extinktionskoeffizienten $E_{1 cm}^{1 \%}$ (280 nm) von 5,9 ± 0,6;
- einem Kohlenhydratanteil von 2,4 ± 0,94 % (g/100 g) (Mannose 0,3 ± 0,2 %, Galactose 0,4 ± 0,2 %, Xylose 0,1 ± 0,04 %, Glukose 0,2 ± 0,1 %, Glukosamin 1,0 ± 0,2 %, Neuraminsäure 0,4 ± 0,2 %) und
- der folgenden Aminosäurenzusammensetzung:

| Aminosäure | Reste pro 100 Reste (Mol %) | Variationskoeffizient |
|---|---|---|
| Lysin | 6,95 | 1,14 |
| Histidin | 0,97 | 17,4 |
| Arginin | 5,44 | 1,77 |
| Asparaginsäure | 11,41 | 1,68 |
| Threonin | 6,78 | 2,40 |
| Serin | 6,21 | 2,26 |
| Glutaminsäure | 12,25 | 0,43 |
| Prolin | 1,96 | 6,20 |
| Aminosäure | Reste pro 100 Reste (Mol %) | Variationskoeffizient |
| Glycin | 6,68 | 3,83 |
| Alanin | 7,92 | 1,67 |
| Cystin 1/2 | 0,77 | 19,5 |
| Valin | 5,34 | 3,80 |
| Methionin | 1,98 | 6,00 |
| Isoleucin | 5,21 | 2,23 |
| Leucin | 11,50 | 0,45 |
| Tyrosin | 3,55 | 4,21 |
| Phenylalanin | 4,07 | 3,77 |
| Tryptophan | 0,93 | 23,9 |

PP4 hemmt die Blutgerinnung reversibel auf der Stufe des Thromboplastins, wie in der deutschen Patentanmeldung P 36 43 182.6 vorgeschlagen und tritt in erhöhten Konzentrationen während und nach Myokardinfarkten sowie bei verschiedenen malignen Tumoren auf.

Wegen der blutgerinnungshemmenden Eigenschaften dieses Proteins sowie des therapeutischen und des diagnostischen Interesses ist eine gentechnische Herstellung äußerst wünschenswert. Die Erfindung betrifft folglich ein Verfahren zur gentechnischen Herstellung von PP4, die dazu erforderliche mRNA, die daraus gewonnene cDNA, diese cDNA ganz oder teilweise enthaltende DNA-Strukturen und Vektoren, mit solcher DNA transformierte Zellen und das von diesen Zellen exprimierte Polypeptid. Die Erfindung bezieht sich weiterhin auf Teilsequenzen der Aminosäuresequenz von PP4, damit gewonnene spezifische Antikörper, aus diesen Antikörpern hergestellte Diagnostika und Antikörpersäulen sowie mit Hilfe solcher Säulen gewonnenes Polypeptid. Ein weiterer Aspekt der Erfindung betrifft Diagnostika, die PP4 codierende bzw. deren komplementäre DNA oder RNA ganz oder teilweise enthalten, und diagnostische Verfahren, mit denen Körperflüssigkeiten und Gewebe mit Hilfe solcher Diagnostika untersucht werden. Weitere Aspekte der Erfindung werden im folgenden näher erläutert bzw. in den Patentansprüchen definiert.

Zur Gewinnung von partiellen Aminosäuresequenzen und daraus abgeleiteten geeigneten Oligonukleotid-Sonden wurde das Protein PP4 in Bromcyanfragmente zerlegt, von denen zwei Fragmente (41 bzw. 44 Aminosäuren) sequenziert wurden. Folgende Sequenzen wurden ermittelt:

PP4-Oligopeptid A

MKGLGTDEES ILTLLTSRSN AQRQEISAAF KTLFGRDLLD D

PP4-Oligopeptid-B

MLVVLLQANR DPDAGIDEAQ VEQDAQALFQ AGELKXGTDE EKFI

Aus dem Oligopeptid A wurden zwei Oligonukleotide von 35 bzw. 36 Basen

ATGAAGGGCC TGGGCACAGA TGAGGAGAGC ATCCT

(PP4-Oligonukleotid 125)

und

CAGGAGATCT CTGCTGCCTT CAAGACCCTG TTTGGC

(PP4-Olignukleotid 197)

und aus dem Oligopeptid B eine Sequenz mit 48 Basen

GACCCTGATG CTGGCATTGA TGAGGCCCAG GTGGAGCAGG ATGCCCAG

(PP4-Oligonukleotid 198)

nach statistischen Daten von R. Lathe (J. Mol. Biol. (1985) 183, 1 - 12) ausgewählt.

Mit diesen Oligonukleotidsonden wurde eine cDNA-Bank einem Screening unterworfen, die aus mRNA von reifer, humaner Plazenta hergestellt worden war. Zunächst wurde aus der Plazenta die mRNA isoliert und daraus die cDNA hergestellt. Diese wurde mit EcoRI-Enden versehen und in die EcoRI-Schnittstelle des Phagenvektors λgt10 ligiert. 46 Klone, die mit den obengenannten Sonden identifiziert worden waren, wurden weiter analysiert. Die Sequenzierung nach an sich bekannten Methoden ergab die DNA-Sequenz, die für PP4 codiert.

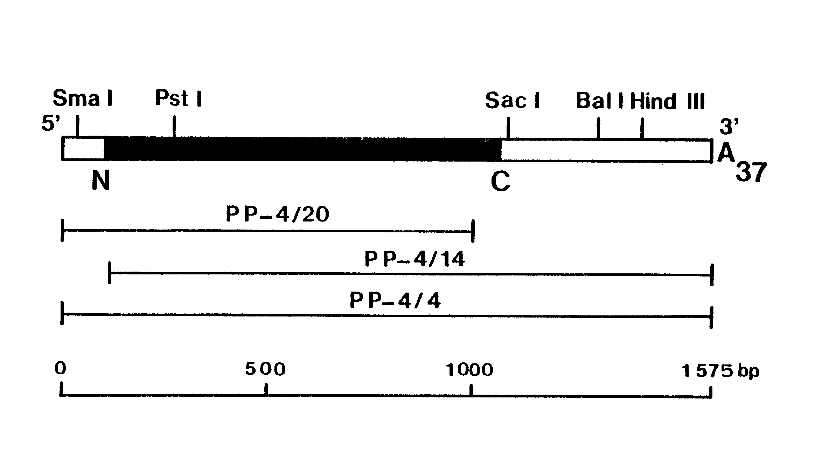

Die Figur zeigt die Restriktionskarte der cDNA-Sequenz, die PP4 codiert. "N" bezeichnet den N-Terminus, "C" den C-Terminus des kodierenden Bereichs, "A (37)" die Poly-(A)-Sequenz von 37 Basen. Die cDNA-Sequenz repräsentiert die gesamte codierende Sequenz von PP4. Die Tabelle 1 zeigt die ermittelte DNA-Sequenz (codierender Strang) und die daraus abgeleitete Aminosäuresequenz eines Klones. Die gesamte cDNA hat eine Länge von 1575 Basenpaaren und einen offenen Leserahmen von 969 Basenpaaren. In der Tabelle 1 sind die Positionen der drei Oligonukleotidsonden markiert, die zwischen den Positionen 188 bis 222, 257 bis 292 bzw. 590 bis 637 hybridisieren.

3

TABELLE 1

```
              10                  30                  50
GCCCCGCGTACCGTCGCCCGGCTCTCCGCCGCTCTCCCGGGGGTTCGGGGCACTTGGGTC

              70                  90                 110
CCACAGTCTGGTCCTGCTTCACCTTCCCCTGACCTGAGTAGTCGCTATGGCACAGGTTCT
                                                  M   A   Q   V   L

             130                 150                 170
CAGAGGCACTGTGACTGACTTCCCTGGATTTGATGAGCGGGCTGATGCACAAACTCTTCG
  R   G   T   V   T   D   F   P   G   F   D   E   R   A   D   A   Q   T   L   R

             190                 210                 230
GAAGGCTATGAAAGGCTTGGGCACAGATGAGGAGAGCATCCTGACTCTGTTGACATCCCG
  K   A   M   K   G   L   G   T   D   E   E   S   I   L   T   L   L   T   S   R

             250                 270                 290
AAGTAATGCTCAGCGCCAGGAAATCTCTGCAGCTTTTAAGACTCTGTTTGGCAGGGATCT
  S   N   A   Q   R   Q   E   I   S   A   A   F   K   T   L   F   G   R   D   L

             310                 330                 350
TCTGGATGACCTGAAATCAGAACTAACTGGAAAATTTGAAAAATTAATTGTGGCTCTGAT
  L   D   D   L   K   S   E   L   T   G   K   F   E   K   L   I   V   A   L   M

             370                 390                 410
GAAACCCTCTCGGCTTTATGATGCTTATGAACTGAAACATGCCTTGAAGGGAGCTGGAAC
  K   P   S   R   L   Y   D   A   Y   E   L   K   H   A   L   K   G   A   G   T

             430                 450                 470
AAATGAAAAAGTACTGACAGAAATTATTGCTTCAAGGACACCTGAAGAACTGAGAGCCAT
  N   E   K   V   L   T   E   I   I   A   S   R   T   P   E   E   L   R   A   I

             490                 510                 530
CAAACAAGTTTATGAAGAAGAATATGGCTCAAGCCTGGAAGATGACGTGGTGGGGGACAC
  K   Q   V   Y   E   E   E   Y   G   S   S   L   E   D   D   V   V   G   D   T

             550                 570                 590
TTCAGGGTACTACCAGCGGATGTTGGTGGTTCTCCTTCAGGCTAACAGAGACCCTGATGC
  S   G   Y   Y   Q   R   M   L   V   V   L   L   Q   A   N   R   D   P   D   A

             610                 630                 650
TGGAATTGATGAAGCTCAAGTTGAACAAGATGCTCAGGCTTTATTTCAGGCTGGAGAACT
  G   I   D   E   A   Q   V   E   Q   D   A   Q   A   L   F   Q   A   G   E   L

             670                 690                 710
TAAATGGGGGACAGATGAAGAAAAGTTTATCACCATCTTTGGAACACGAAGTGTGTCTCA
  K   W   G   T   D   E   E   K   F   I   T   I   F   G   T   R   S   V   S   H

             730                 750                 770
TTTGAGAAAGGTGTTTGACAAGTACATGACTATATCAGGATTTCAAATTGAGGAAACCAT
  L   R   K   V   F   D   K   Y   M   T   I   S   G   F   Q   I   E   E   T   I

             790                 810                 830
TGACCGCGAGACTTCTGGCAATTTAGAGCAACTACTCCTTGCTGTTGTGAAATCTATTCG
  D   R   E   T   S   G   N   L   E   Q   L   L   L   A   V   V   K   S   I   R

             850                 870                 890
AAGTATACCTGCCTACCTTGCAGAGACCCTCTATTATGCTATGAAGGGAGCTGGGACAGA
  S   I   P   A   Y   L   A   E   T   L   Y   Y   A   M   K   G   A   G   T   D
```

```
           910                    930                    950
TGATCATACCCTCATCAGAGTCATGGTTTCCAGGAGTGAGATTGATCTGTTTAACATCAG
  D   H   T   L   I   R   V   M   V   S   R   S   E   I   D   L   F   N   I   R

           970                    990                   1010
GAAGGAGTTTAGGAAGAATTTTGCCACCTCTCTTTATTCCATGATTAAGGGAGATACATC
  K   E   F   R   K   N   F   A   T   S   L   Y   S   M   I   K   G   D   T   S

          1030                   1050                   1070
TGGGGACTATAAGAAAGCTCTTCTGCTGCTCTGTGGAGAAGATGACTAACGTGTCACGGG
  G   D   Y   K   K   A   L   L   L   L   C   G   E   D   D

          1090                   1110                   1130
GAAGAGCTCCCTGCTGTGTGCCTGCACCACCCCACTGCCTTCCTTCAGCACCTTTAGCTG

          1150                   1170                   1190
CATTTGTATGCCAGTGCTTAACACATTGCCTTATTCATACTAGCATGCTCATGACCAACA

          1210                   1230                   1250
CATACACGTCATAGAAGAAAATAGTGGTGCTTCTTTCTGATCTCTAGTGGAGATCTCTTT

          1270                   1290                   1310
GACTGCTGTAGTACTAAAGTGTACTTAATGTTACTAAGTTTAATGCCTGGCCATTTTCCA

          1330                   1350                   1370
TTTATATATATTTTTTAAGAGGCTAGAGTGCTTTTAGCCTTTTTTAAAAACTCCATTTAT

          1390                   1410                   1430
ATTACATTTGTAACCATGATACTTTAATCAGAAGCTTAGCCTTGAAATTGTGAACTCTTG

          1450                   1470                  ·1490
GAAATGGTATTAGTGAAGTTCGCAACTAAACTAAACCTGTAAAATTATGATGATTGTATT

          1510                   1530                   1550
CTTTAGATTAATGAAAAATAAACATTTCTGTCCCCCTGAAAAAAAAAAAAAAAAAAAAAA

          1570
AAAAAAAAAAAAAA
```

Erfindungsgemäß kann die kodierende cDNA mittels geeigneter Expressionssysteme dazu benutzt werden, PP4 zu exprimieren. Durch die Auswahl des Wirtes kann weiterhin die Form der Modifikation von PP4 beeinflußt werden. So findet in Bakterien keine, in Hefezellen eine andere Glykosylierung als in höheren eukaryotischen Zellen statt.

In Kenntnis der Aminosäuresequenz von PP4 ist es möglich, nach konventionellen oder gentechnischen Methoden Aminosäure-Teilsequenzen herzustellen, die als Antigene zur Herstellung von polyklonalen oder monoklonalen Antikörpern dienen können. Solche Antikörper können nicht nur zu diagnostischen Zwecken, sondern auch zur Herstellung von Antikörpersäulen dienen, mit denen PP4 aus Lösungen abgetrennt werden kann, die es neben anderen Proteinen enthalten.

Mit Hilfe der cDNA bzw. Teilen davon kann man auch auf einfache Weise aus einer genomischen Bank den für PP4 codierenden genomischen Klon isolieren, mit dessen Hilfe nicht nur eine Expression in eukaryotischen Zellen erleichtert wird, sondern auch weitere diagnostische Aussagen getroffen werden können.

Die Erfindung ist ferner in den Patentansprüchen definiert und in folgenden Beispielen weiter ausgeführt.

Soweit nicht im Text erläutert, werden die folgenden Abkürzungen verwendet:

| EDTA | = Natrium-ethylendiamin-tetraacetat |
| SDS | = Natrium-dodecylsulfat |
| DTT | = Dithiothreitol |
| BSA | = Rinderserumalbumin |

**Beispiele:**


1. Isolierung von RNA aus humaner Plazenta

RNA wurde aus reifer, humaner Plazenta (nach Chirgwin et al., Biochemistry 18 (1979) 5294-5299) gewonnen. Etwa 10 g Plazentagewebe wurden in flüssigem Stickstoff zermörsert, in 80 ml 4 M Guanidinium-Thiocyanat mit 0,1 M Mercaptoethanol suspendiert und 90 sec. bei 20 000 rpm in einem Homogenisator (Ultraturrax) behandelt. Das Lysat wurde 15 min. bei 7 000 rpm zentrifugiert (Sorvall-GSA Rotor) und der Überstand mit 2 ml 1 M Essigsäure und 60 ml Ethanol abs. bei -20° C über Nacht gefällt. Nach Sedimentation bei 6 000 rpm und -10° C für 10 min. wurden die Nucleinsäuren in 40 ml 7,5 M Guanidinium-Hydrochlorid (pH 7,0) vollständig gelöst und mit einer Mischung aus 1 ml 1 M Essigsäure und 20 ml Ethanol abs. gefällt. Zur Abtrennung der DNA wurde die Fällung ein weiteres Mal mit jeweils halben Volumina wiederholt. Die RNA wurde in 12 ml $H_2O$ gelöst, mit einer Mischung aus 1,2 ml 4 M Kaliumacetat und 24 ml Ethanol abs. gefällt, sedimentiert und schließlich erneut in 10 ml $H_2O$ (1 ml pro g Gewebe) aufgenommen.


2. Gewinnung von Poly(A)-haltiger Plazenta-mRNA

Die Plazenta-RNA wurde zur Gewinnung von Poly(A)-haltiger mRNA über Oligo(dT)-Cellulose-Chromatographie (Aviv and Leder, Proc. Natl. Acad. Sci. USA 69 (1973) 1408-1412) in 2 ml Pasteurpipetten in LiCl aufgetrennt. Etwa 5 mg Plazenta-RNA wurden in Puffer 1 (500 mM LiCl, 20 mM Tris (pH 7,5), 1 mM EDTA, 0,1 % SDS) auf die Säule aufgetragen. Während die Poly(A)$^+$-RNA an Oligo(dT)-Cellulose gebunden wurde, konnte die Poly(A)$^-$-RNA wieder eluiert werden. Nach einem Waschschritt mit Puffer 2 (100 mM LiCl, 29 mM Tris (pH 7,5), 1 mM EDTA, 0,1 % SDS) wurde die Poly(A)$^+$-RNA (Plazenta-mRNA) mit Puffer 3 (5 mM Tris (pH 7,5), 1 mM EDTA, 0,05 % SDS) von der Säule eluiert.

Zur weiteren Reinigung wurde due Poly(A)$^+$-RNA auf Puffer 1 eingestellt und erneut über Oligo(dT)-Cellulose chromatographiert.

Die Ausbeute an Plazenta Poly(A)$^+$-RNA betrug nach diesem zweiten Reinigungsschritt etwa 4 % der eingesetzten RNA.


3. Synthese von cDNA aus humaner Plazenta (Plazenta-cDNA) und doppelsträngiger cDNA (dsDNA)

Poly(A)-haltige Plazenta-mRNA wurde vor der cDNA-Synthese im 1,5 % Agarosegel auf Intaktheit geprüft.

Danach wurden 4 μg Plazenta-mRNA in 65,5 μl $H_2O$ gelöst, 10 min. be

Die cDNA-Synthese erfolgte in einem 100 μl-Ansatz nach Zugabe von 20 μl RT$_1$-Puffer (250 mM Tris (pH 8,2) bei 42° C, 250 mM KCl, 30 mM $MgCl_2$), 2,5 μl 20 mM dNTP (d.h. aller vier Desoxynukleosidtriphosphate), 1 μl Oligo(dT) von 1 μg/ml, 1 μl 1 M DTT, 2 μl RNAsin und 8μl Reverse Transcriptase (24 U/μl) für 90 min. bei 42° C. Doppelsträngige cDNA (dsDNA) wurde nach Gubler and Hoffmann (Gene 25 (1983) 263-269) synthetisiert. Die Synthese erfolgte unmittelbar nach der cDNA-Synthese durch Zugabe von 305,5 μl $H_2O$, 80 μl RT$_2$-Puffer (100 mM Tris (pH 7,5), 25 mM $MgCl_2$, 500 mM KCl, 50 mM DTT, 250 μg/ml BSA), 2 μl RNase H (2 U/μl), 2,5 μl E. coli DNA Ligase (5 U/μl), 5 μl 15 mM β-NAD, und 5 μl DNA Polymerase I (5 U/μl) und Inkubation für 5 h bei 15° C. Durch Hitzeinaktivierung (70° C, 30 min.) wurde die Reaktion beendet.

Der Reaktionsansatz wurde nach Zugabe von 55 μl 250 μM dNTP, 55 μl 10 mM Tris (pH 7,5), 10 mM $MgCl_2$, 10 μg/ml BSA, 3 μl T4 DNA-Polymerase I (1 U/μl), 2 μl RNase H (2 U/μl) und 2 μl RNase A (2 μg/ml) für weitere 30 min. bei 37° C inkubiert, um die Vollständigkeit der Synthese am zweiten DNA-Strang zu gewährleisten ("Repair Reaction").

4. Ligierung von EcoRI-Linkern an die dsDNA und Öffnen der Linker

Zur Errichtung einer Plazenta-cDNA-Bank wurde die dsDNA mit EcoRI-Enden versehen, um sie in die EcoRI-Schnittstelle des Phagenvektors λgt10 ligieren zu können (T. Maniatis et al. (1982), Molecular Cloning, A Laboratory Manual, Cold Spring Harbor). Hierzu wurde die dsDNA

    a) mit EcoRI-Methylase behandelt, um interne EcoRI-Schnittstellen der dsDNA zu schützen,
    b) mit EcoRI-Linkern versehen, die
    c) danach mit EcoRI geöffnet wurden.

Zu a):

Die Methylase-Reaktion der dsDNA erfolgte direkt im Anschluß an die "Repair Reaction" nach Zugabe von 25 µl 500 mM EDTA (pH 8,0), 60 µl Methylase-Puffer (100 mM NaOAc (pH 5,2), 2 mg S-Adenosyl-L-Methionin) und 2 µl EcoRI-Methylase (20 U/µl) durch Inkubation bei 37°C für 30 min. Der Reaktionsansatz wurde mit Phenol extrahiert und die dsDNA mit 60 µl 4M NaOAc und 1300 µl Ethanol gefällt. Die dsDNA wurde zweimal mit 70 % Ethanol gewaschen, einmal mit Ether ausgeschüttelt und getrocknet.

Zu b):

Die EcoRI-methylierte dsDNA wurde in 88 µl $H_2O$ gelöst und nach Zugabe von 10 µl Ligase-Puffer (500 mM Tris (pH 7,4), 100 mM $MgCl_2$, 100 mM DTT, 10 mM Spermidin, 10 mM ATP, 1 mg/ml BSA), 1 µl T4 DNA-Ligase (10 U/µl) mit 1 µl EcoRI Linkern (0,5 µg/µl) (pGGAATTCC bzw. pAGAATTCT) über Nacht bei 15°C ligiert.

Zu c):

Das Volumen des Ligase-Ansatzes wurde mit 6 µl $H_2O$, 12 µl 10x EcoRI-Puffer und 2 µl EcoRI (120 U/µl) auf 120 µl gebracht. Die EcoRI-Verdauung erfolgte für 2 h bei 37°C.

5. Abtrennen nichtgebundener Linker über Kaliumacetat-Gradienten und Größenselektionierung der dsDNA

Zur Abtrennung aller nichtgebundenen EcoRI-Linker von der dsDNA wurde der EcoRI-Reaktionsansatz in toto auf einen Kaliumacetat-Gradienten (5-20 % KOAc, 1 mM EDTA, 1 µl/ml Ethidiumbromid) aufgetragen und 3 h bei 50 000 rpm und 20°C zentrifugiert (Beckman SW 65-Rotor). Der Gradient wurde von unten so fraktioniert, daß die ersten fünf Fraktionen 500 µl maßen und alle restlichen 100 µl. Die Fraktionen wurden mit 0,01 Volumen Acrylamid (2 mg/ml) und 2,5 Volumen Ethanol gefällt, einmal mit 70 %igem Ethanol gewaschen, getrocknet und jeweils in 5 µl $H_2O$ aufgenommen. Zur Größenbestimmung der dsDNA wurden 1 µl jeder Fraktion im 1,5 % Agarose-Gel analysiert. Zusätzlich wurde mit 1 µl jeder Fraktion die Quantität an dsDNA bestimmt. Fraktionen mit dsDNA über 1000 bp wurden vereinigt und die Probe so eingeengt, daß die Endkonzentration 27 µg/ml betrug.

6. Einbau der dsDNA in den Phagenvektor λgt10 und "in vitro packaging"-Reaktion

Der Einbau der dsDNA in die EcoRI-Schnittstelle des Phagenvektors λgt10 (Vector Cloning Systems, San Diego, CA) erfolgte in einem Ligaseansatz von 4 µl: 2 µl dsDNA, 1 µl λgt10 x EcoRI (1 µg/ml), 0,4 µl Ligase-Puffer, 0,5 µl $H_2O$, 0,1 µl T4 DNA-Ligase. Der Ansatz wurde 4 h bei 15°C inkubiert. Zur Etablierung der Plazenta-cDNA-Bank im Phagenvektor λgt10 folgte mit den λ-lysogenen Zellextrakten E. coli NS 428 und NS 433 eine "in vitro packaging"-Reaktion des Ligaseansatzes bei Raumtemperatur für 2 h (Vector Cloning Systems, San Diego, CA; Enquist and Sternberg, Methods in Enzymology 68, (1979), 281-298). Die Reaktion wurde mit 500 µl Suspensionsmedium (SM: 0,1 M NaCl, 8 mM MgSO₄, 50 mM Tris (pH 7,5), 0,01 % Gelatine) und 2 Tropfen Chloroform gestoppt.

### 7. Titerbestimmung und Analyse der Plazenta-cDNA-Bank

Die Zahl der "Plaque Forming Units" (PFU) der Plazenta-cDNA-Bank wurde mit kompetenten Zellen des E. coli- K 12-Stammes C600 HFL bestimmt: Sie betrug $1 \times 10^6$ PFU. Etwa 80 % aller Phagen enthielten DNA-Inserts, die größer als 1000 Basenpaare waren.

### 8. Oligonukleotidsonden zum "Screening" der Plazenta-cDNA-Bank

Drei Oligonukleotidsonden wurden zur Analyse der Plazenta-cDNA-Bank synthetisiert. Ihre Sequenzen wurden abgeleitet von der Aminosäuresequenz zweier Bromcyanfragmente von PP4.

Die Art des Aufbaus und die Verwendung beider Sonden folgten im wesentlichen den Regeln von R. Lathe, a.a.O..

Die drei Oligonukleotidsequenzen wurden mit T4 Polynukleotidkinase unter Anwesenheit von ($\gamma$-32P)-ATP am 5′-Ende markiert (ca. 1 µg DNA ($\gamma$-32P)ATP:3000Ci/mmol, 10 µCi/µl, eingesetzt wurden 6 µl/40 µl Reaktionsansatz). Die Sonden hatten eine spezifische Aktivität von $1 \times 10^8$ Bq/µl bzw. $1,5 \times 10^6$ Bq/pMol.

### 9. "Screening" der Plazenta-cDNA mit PP4 spezifischen Oligonukleotiden

$1 \times 10^6$ PFU der Plazenta-cDNA-Bank wurden mit den PP4-Oligonukleotidsonden 125, 197 und 198 zusammen untersucht. Dazu wurden $3 \times 10^4$ PFU mit Zellen des E. coli K 12-Stammes C 600 HFL in Weich-Agar auf 13,5 cm Petrischalen ausplattiert und 6 h bei 37° C inkubiert. Zu diesem Zeitpunkt war noch keine vollständige Lyse eingetreten. Die Platten wurden über Nacht im Kühlschrank inkubiert und die Phagen auf Nitrocellulosefilter (Schleicher & Schüll, BA 85, Ref.-Nr. 401124) übertragen (Duplikate). Nitrocellulosefilter und Petrischalen wurden mit einer Injektionskanüle markiert, um eine spätere Zuordnung zu ermöglichen. Die Petrischalen wurden während des Prozessierens der Nitrocellulosefilter im Kühlraum gelagert. Die auf den Nitrocellulosefiltern befindliche DNA wurde denaturiert, indem die Filter für 5 min. auf mit 1,5 M NaCl, 0,5 M NaOH-getränktes Filterpapier (Whatman-M3) gelegt wurden. Anschließend wurden die Filter auf die gleiche Art mit 1,5 M NaCl, 0,5 M Tris (pH 8,0) renaturiert une mit 2 x SSPE (0,36 M NaCl, 16 mM NaOH, 20 mM NaH₂PO₄, 2 mM EDTA) gewaschen. Die Filter wurden dann für 2 h bei 80° C im Vakuum getrocknet. Die Filter wurden für 4 h bei 65° C in 3xSSC, 0,1 % SDS (20 x SSC = 3 M NaCl, 0,3 M Na-Citrat) gewaschen und für 4 h bei 65° C vorhybridisiert (Prähybridisierungslösung: 0,6 M NaCl, 0,06 M Tris (pH 8,3), 6 mM EDTA, 0,2 % nichtionisches synthetisches Saccharose-Polymer (RFicoll), 0,2 % Polyvinylpyrrolidon 40, 0,2 % BSA, 0,1 % SDS, 50 µg/ml denaturierte Heringssperma-DNA). Die Filter wurden über Nacht unter Zusatz von 100 000-200 000 Bq des markierten Oligonukleotids/ml Hybridisierungslösung (wie Prähybridisierungslösung, jedoch ohne Heringssperma-DNA) in Bechergläsern bzw. in zugeschweißten Polyethylenfolien unter leichtem Schütteln inkubiert. Die Hybridisierungstemperatur betrug 44° C bzw. 52° C. Die Nitrocellulosefilter wurden mit 6 x SSC, 0,05 M Natriumpyrophosphat eine Stunde bei Raumtemperatur und für eine weitere Stunde bei der jeweiligen Hybridisierungstemperatur gewaschen. Die Filter wurden getrocknet und über Nacht autoradiographiert. Signale auf dem Röntgenfilm, die auf beiden Duplikaten auftraten, wurden der Petrischale zugeordnet und die Region (ca. 50 Plaques) mit dem breiten Ende einer Pasteurpipette ausgestanzt und die Phagen in 1 ml SM-Puffer resuspendiert. Positive Phagen wurden über drei Runden vereinzelt, bis ein einzelner Klon vorlag.

Insgesamt wurden $1 \times 10^6$ PFU der Plazenta-cDNA-Bank untersucht. Es wurden 54 Signale auf Duplikat-Filtern identifiziert. Beim weiteren Screening mit den einzelnen Sonden zeigte sich, daß 18 Klone mit allen Sonden reagierten. Alle diese 18 Klone tragen eine PP4 kodierende Sequenz mit einer maximalen Länge von 1575 Basenpaaren für den Klon PP4/4.

Der Vergleich der Oligonukleotidsequenzen 125, 197 und 198 mit der ermittelten PP4-Sequenz ist in Tabelle 2 zusammengefaßt.

**Tabelle 2**

PP4-Sequenz vs. PP4-Oligonukleotid 125

```
          .           .           .           .           .
151 TGATGAGCGGGCTGATGCACAAACTCTTCGGAAGGCTATGAAAGGCTTGG 200
                                           | | | | - | | | - | | |
  1 .................................................ATGAAGGGCCTGG 13
```

```
          .           .           .           .           .
201 GCACAGATGAGGAGAGCATCCTGACTCTGTTGACATCCCGAAGTAATGCT 250
    | | | | | | | | | | | | | | | | | | |
 14 GCACAGATGAGGAGAGCATCCT............................ 35
```

PP4-Sequenz vs. PP4-Oligonukleotid 197

```
          .           .           .           .           .
251 CAGCGCCAGGAAATCTCTGCAGCTTTTAAGACTCTGTTTGGCAGGGATCT 300
        | | | | - | | | | | | | - | | - | | - | | | | | | | | | | | | |
  1 ......CAGGAGATCTCTGCTGCCTTCAAGACCCTGTTTGGC........ 36
```

PP4-Sequenz vs. PP4-Oligonukleotid 198

```
          .           .           .           .           .
551 TACCAGCGGATGTTGGTGGTTCTCCTTCAGGCTAACAGAGACCCTGATGC 600
                                              | | | | | | | | | |
  1 ..........................................GACCCTGATGC 11
```

```
          .           .           .           .           .
601 TGGAATTGATGAAGCTCAAGTTGAACAAGATGCTCAGGCTTTATTTCAGG 650
    | | | - | | | | | | | | - | | - | | - | | - | | | | | - | | |
 12 TGGCATTGATGAGGCCCAGGTGGAGCAGGATGCCCAG............. 48
```

10. DNA-Sequenzanalyse

Die Phagenklone PP4/20, PP4/14 und PP4/4 wurden vermehrt und ihre DNA jeweils extrahiert. Das jeweilige EcoRI-Fragment wurde isoliert und in die EcoRI-Stelle des Bluescript M13 Vektors (Stratagene, San Diego, CA, USA) für Restriktionsanalysen und Sequenzanalysen mittels der enzymatischen Dideoxymethode nach Sanger einligiert. Die Sequenz zeigt einen offenen Leserahmen und kodiert für ein Protein mit maximal 320 Aminosäuren.

11. Expression des anticoagluatorischen Proteins PP4

pTrc98-1 (Europäische Patentanmeldung EP 0 236 978) wurde mit BglII linarisiert und die überlappenden Enden mittels Klenow-Polymerase aufgefüllt. Die DNA wurde anschließend mit Phosphatase behandelt. Das Plasmid placI<sup>q</sup> (Wang et al. (1983) Cold Spring Harbor Symp. Quan. Biol. 47, 85-91) wurde mit EcoRI verdaut, die überlappenden Enden ebenfalls mittels Klenow Polymerase aufgefüllt und das ca. 1100 Basenpaar große Fragment, welches das vollständige lacI<sup>q</sup> Allel trägt (Calos (1978) Nature 274, 762-765), wurde mit der linearisierten pTrc98-1 DNA ligiert. Das resultierende Plasmid (mit der Transcriptionsrichtung des lacI<sup>q</sup> Genes identisch zur Transcriptionsrichtung des trc Promoters) war pTrc90-3. Die Sequenz von pTrc90-3 umfaßt 4134 Basenpaare. pTrc90-3 wurde mit NcoI und HindIII verdaut und das große Fragment mit den synthetischen Oligonukleotiden A, B oder C in drei getrennten Ansätzen ligiert und anschließend in kompetente Zellen des E.coli K12 Stammes W3110lacI<sup>q</sup> transformiert:

```
                              5' CATGGAATTCCGA
        Oligo A                  |||||||||
                                 CTTAAGGCTTCGA 5'


                              5' CATGGGAATTCGA
        Oligo B                  |||||||||
                                 CCTTAAGCTTCGA 5'


                              5' CATGGGGAATTCGA
        Oligo C                  ||||||||||
                                 CCCTTAAGCTTCGA 5'
```

Die resultierenden Plasmide pTrc98A (Oligo A); pTrc98B (Oligo B) und pTrc98C (Oligo C) wurden mit EcoRI und HindIII verdaut, das große Fragment jeweils isoliert und mit dem 55 Basenpaar großen EcoRI-HindII Polylinkerfragment des handelsüblichen Vektors pUC18 (Yanisch-Perron et al. (1985) Gene 33, 103-119) ligiert. Die resultierenden Plasmide sind pTrc99A (4176 Basenpaare), pTrc99B (4177 Basenpaare) und pTrc99C (4178 Basenpaare). Als Folge der einligierten sythetischen Oligonukleotide A, B und C unterscheiden sich die Vektoren um jeweils eine Base zwischen der NcoI und der EcoRI Erkennungsstelle, was wiederum in einem Verschieben des Translationsleserasters resultiert. Die Anwesenheit des lacI<sup>q</sup> Gens auf dem Expressionsvektor (pTrc99 Serie) ist dann interessant, wenn E.coli Stämme zur Expression benutzt werden sollen, die keinen endogenen lac Repressor besitzen, bzw. in solchen Fällen, in denen das exprimierte Protein toxische Wirkung auf die E.coli Wirtszelle ausübt und aus diesem Grunde eine vollständige Repression in der Klonierungs bzw. Anzuchtphase erforderlich ist.

Im vorliegenden Ausführungsbeispiel wurde der Vektor pTrc99A zur Expression des unfusionierten, reifen PP4 Proteins in E.coli benutzt. Die DNA Sequenz der PP4 cDNA am Initiationscodon lautet:

```
                    Met Ala Gln Val
        5'...GTAGTCGCT ATG GCA CAG GTT ... 3'
```

Da keine NcoI Stelle am ATG vorhanden ist, kann diesen DNA nicht direkt in den pTrc99A Expressionsvektor kloniert werden. Eine NcoI Stelle kann aber durch einen einzigen Basenaustausch (von "T" nach "C") in der PP4 cDNA Sequenz: 5' CTATGG 3' nach 5' CCATGG 3' erzielt werden. die zweite Aminosäure (Ala) ist von dieser Manipulation nicht betroffen, da das zweite Codon der PP4 Struktursequenz mit einem "G" beginnt. Zur Mutagenese wurde ein 1462 Basenpaar großes EcoRI-HindIII Fragment des PP4 Klons PP4-4 isoliert und in den ebenfalls mit EcoRI und HindIII geschnittenen Mutagenesevektor pMa5-8 ligiert. Der Mutagenesevektor pMa 5-8 trägt neben einem bakteriellen Replikationsorigin und einem Antibiotikaresistenzmarker eine Klonierungs-Polylinkerregion und den Replikationsorigin des einzelsträngigen Bakteriopha-

,gen Fl. Letzteres führt dazu, daß ein Einzelstrang der klonierten PP4 cDNA nach bekannten Methoden isoliert und dem publizierten "gapped-Duplex" Mutageneseprotokoll (Kramer et al. (1984) Nucl. Acids Res. 12, 9441-9456) unterzogen werden kann, wobei folgendes Oligodesoxynucleotid verwendet wurde:

5′ GAACCTGTGCCATGGCGACTACTCTAGG 3′

Ein Klon, der die gewünschte NcoI-Mutation aufwies, wurde durch entsprechende Restriktionsananlyse identifiziert und als pMc5-8-PP4-NcoI bezeichnet. Aus diesem Plasmid wurde das 1305 Basenpaar große NcoI-HindIII Fragment isoliert und in den entsprechend geschnittenen pTrc99A Vektor ligiert. Das resultierende Plasmid pTrc99A-PP4 umfaßt 5425 Basenpaare und exprimiert nach Induktion des trc Promoters das unfusionierte, ca. 35 kD große PP4 Protein. Das Protein macht ca. 5% am totalen zellulären Protein aus und ist löslich. Es reagiert spezifisch mit Anti-PP4-Antiseren, jedoch nicht mit Anti-PP4-X-Antiserum.

**Ansprüche**

1. DNA-Sequenz, kodierend für anticoagulatorisches Protein PP4, enthaltend den kodierenden Strang gemäß Tabelle 1.
2. DNA oder RNA, die mit der DNA gemäß Anspruch 1 unter stringenten Bedingungen hybridisiert.
3. DNA-Sequenz, kodierend für die Aminosäuresequenz gemäß Tabelle 1.
4. Genstruktur, enthaltend eine DNA gemäß Anspruch 1, 2 oder 3.
5. Vektor, enthaltend eine DNA nach einem oder mehreren der vorhergehenden Ansprüche.
6. Transformierte Zelle, enthaltend DNA nach Anspruch 1, 2, 3 oder 4.
7. Gentechnisch erhältliches PP4, gekennzeichnet durch die Aminosäuresequenz gemäß Tabelle 1.
8. Verfahren zur Herstellung von PP4, dadurch gekennzeichnet, daß man eine cDNA nach Anspruch 1, 3 oder 4, wobei sich Anspruch 4 nur auf Anspruch 1 und 3 bezieht, in ein Expressionssystem einbringt und dort zur Expression bringt.
9. Für PP4 spezifische polyklonale oder monoklonale Antikörper, erhalten aus gentechnisch hergestelltem PP4 bzw. antigenwirksamen Teilen davon.
10. Diagnostikum, das eine DNA nach Anspruch 1, 2, 3 oder 4 ganz oder teilweise enthält.
11. Diagnostikum, das eine DNA oder RNA ganz oder teilweise enthält, die zu der DNA nach Anspruch 1 komplementär ist.
12. Diagnostikum, enthaltend Antikörper nach Anspruch 9.
13. Diagnostizierverfahren, dadurch gekennzeichnet, daß Körperflüssigkeiten, Gewebe oder daraus isolierte Nukleinsäuren mit einem Diagnostikum nach Anspruch 10, 11 oder 12 in Kontakt gebracht werden.
14. Arzneimittel, gekennzeichnet durch einen Gehalt an nach Anspruch 8 hergestelltem PP4.

Patentansprüche für folgende Vertragsstaaten: ES, GR

1. Verfahren zur Herstellung von anticoagulatorischem Protein PP4, dadurch gekennzeichnet, daß die kodierende DNA-Sequenz gemäß Tabelle 1 in geeignete Expressionsvektoren gebracht wird und in geeigneten Expressionssystemen exprimiert wird.
2. Verfahren zur Herstellung von polyklonalen oder monoklonalen Antikörpern, dadurch gekennzeichnet, daß das nach Anspruch 1 hergestellte PP4 oder antigenwirksame Teile davon zur Immunisierung eingesetzt wird.
3. Verfahren zur herstellung von Diagnostika, dadurch gekennzeichnet, daß eine Nukleinsäuresequenz nach Tabelle 1, Teile davon oder dazu komplementäre Sequenzen eingesetzt werden.
4. Verfahren zur Herstellung eines Diagnostikums, dadurch gekennzeichnet, daß nach Anspruch 2 hergestellte Antikörper eingesetzt werden.
5. Diagnostizierverfahren, dadurch gekennzeichnet, daß Körperflüssigkeiten, Gewebe oder daraus isolierte Nukleinsäuren mit einem Diagnostikum hergestellt nach Anspruch 3 oder 4 in Kontakt gebracht werden.
6. Verfahren zur Herstellung eines Arzneimittels, dadurch gekennzeichnet, daß nach Anspruch 1 hergestelltes PP4 eingesetzt wird.

11

Sma I    Pst I                                    Sac I    Bal I  Hind III

5'  |    |                                        |        |      |        3'

N                                                 C                         A 37

PP–4/20

PP–4/14

PP–4/4

0                    500                1000                  1575 bp

EP 0 318 703 A1

**Europäisches Patentamt**

# EUROPÄISCHER RECHERCHENBERICHT

Nummer der Anmeldung

EP 88 11 8039

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,X | CHEMICAL ABSTRACTS Band 108, Nr. 13, 28. März 1988, Seite 152, Zusammenfassung Nr. 107278x, Columbus, Ohio, US; A. IWASAKI et al.: "Structure and expression of cDNA for an inhibitor of blood coagulation isolated from human placenta: a new lipocortin-like protein" & J. Biochem.(Tokyo), Band 102, Nr. 5, 1987, Seiten 1261 - 1273 --- | 1-8 | C 12 N 15/00 |
| P,X | CHEMICAL ABSTRACTS Band 108, Nr. 1, 4. Januar 1988, Seite 230, Zusammenfassung Nr. 2346q, Columbus, Ohio, US; T. FUNAKOSHI et al.: "Primary structure of human placental anticoagulant protein." & Biochemistry, Band 26, Nr. 25, 1987, Seiten 8087 - 8092 --- | 1-7 | |
| X | CHEMICAL ABSTRACTS Band 105, Nr. 15, 13. Oktober 1986, Seite 335, Zusammenfassung Nr. 130108p, Columbus, Ohio, US; S. SHIROTAKE et al.: "Immunochemical measurement of placental tissue protein 4 in serum." & Chem. Pharm. Bull., Band 34, Nr. 5, 1986, Seiten 2191 - 2195 --- | 9,12,13 | RECHERCHIERTE SACHGEBIETE (Int. Cl.4) C 12 N 15/00 |
| P,X | EP-A-0 271 885 (BEHRINGWERKE) * Seite 3, Zeilen 34 - 42; Seite 3, Zeile 49; Ansprüche * & DE - A - 36 43182 (Cat. D) --- | 7,14 | |
| X | EP-A-0 123 307 (BEHRINGWERKE) * insgesamt * & DE - A - 33 15000 (Cat. D) --- | 7,9,12, 13 | |

-/-

Der vorliegende Recherchenbericht wurde für alle Patentansprüche erstellt

| Recherchenort | Abschlußdatum der Recherche | Prüfer |
|---|---|---|
| BERLIN | 24-01-1989 | JULIA P. |

**Europäisches Patentamt**

**·EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | --- | 1-6,8 | |
| Y | EP-A-0 150 735 (CHIRON CORPORATION) * insgesamt * | 1-6,8 | |
| | --- | | |
| A | EP-A-0 217 341 (KOWA CO LTD) * insgesamt * | 1 | |
| | --- | | |
| E | PATA BIOSIS Zusammnfassung Nr. 86058664; U. GRUNDMANN et al.: "Characterization of complementary DNA encoding human placental anticoagulant protein PP4 homolgy with the lipocortin family" & Proc. Natl. Acad. Sci., US, Band 85, Nr. 11, 1988, Seiten 3708 - 3712 ----- | 1-7 | |

**RECHERCHIERTE SACHGEBIETE (Int. Cl.4)**

Der vorliegende Recherchenbericht wurde für alle Patentansprüche erstellt

| Recherchenort | Abschlußdatum der Recherche | Prüfer |
|---|---|---|
| BERLIN | 24-01-1989 | JULIA P. |

EPO FORM 1503 03.82 (P0403)